# EUROPEAN PATENT APPLICATION

(11) **EP 2 612 670 A2**
(43) Date of publication of application: **10.07.2013**
(21) Application number: 11820941.0
(22) Date of filing: 05.09.2011
(51) Int. Cl.: A61K 31/5517, A61K 31/135, A61P 23/00, A61P 25/20, A61P 25/04

(54) **ANESTHETIC ASSOCIATION, ANESTHETIC COMPOSITION, AND ANESTHETIC MEDICAMENT FOR VETERINARY USE**

(30) Priority: 03.09.2010 BR PI1003620
(71) Applicant: Ouro Fino Participações E Empreendimentos S/A., 14025-230 BR Ribeirão Preto - SP (BR)
(72) Inventor: CARLOS ENRIQUE, Enrique, CEP: 14098-558 Ribeirão Preto SP (BR)
(74) Representative: Eterno, Enrico
(86) International application number: PCT/BR2011/000312
(87) International publication number: WO 2012/027811

(57) **Abstract**

The present invention relates to a new association of active ingredients working as anesthetic and pre-anesthetic formulated in a composition containing anesthetics and analgesics suitable for use in surgical and pre-surgical procedures and others which require sedation, tranquilization, chemical restraint and pre-anesthetic medication. More specifically the present invention relates to a new association comprising the active ingredients ketamine, preferably the isomer S-ketamine(+), midazolam and tramadol, formulated in true solution for parenteral use. The present invention also contemplates an anesthetic drug for veterinary use for parenteral use.

## Description

### Field of the Invention

The present invention relates to a new association of active ingredients working as anesthetic and pre-anesthetic formulated in a composition containing anesthetics and analgesics especially suitable for use in surgical and pre-surgical procedures and others which require sedation, tranquilization, chemical restraint and pre-anesthetic medication.

More specifically, the present invention relates to a new association comprising the active ingredients ketamine, midazolam, and tramadol, formulated in true solution for parenteral use intended for veterinary anesthesia and analgesia. More preferably, ketamine is the isomer S-ketamine (+).

The present invention also contemplates an anesthetic drug for veterinary use.

Several drugs are used as pre-anesthetic medication having as some of its main goals the reduction of the dose of the inducing agent and the production of a preventive analgesia. Among those drugs are dissociative agents, benzodiazepines and opioids.

Ketamine is a phencyclidine derivative that acts by inhibition mechanisms of the corticothalamic system and concomitant activation of the limbic system. It produces dissociative-type anesthesia of short duration, characterized by the absence of responses to nociceptive stimuli and maintenance of protective reflexes. Ketamine, by concomitantly increasing the heart rate, cardiac output, blood pressure, left ventricular work and myocardial oxygen consumption, has been associated to sedatives or tranquilizers that eliminate or minimize such effects. Currently, the availability of the isomer S-ketamine (+) aroused great interest since it shows power two times greater than the racemic mixture, but a lesser amount of drug is required to produce the same analgesic effects with lower incidence of adverse effects than the racemic formulation, decreasing the severity of ischemic brain lesions by different mechanisms.

Ketamine, enantiomers and salts thereof and processes for their preparation are referenced and disclosed in U.S. Patent 6,743,949, corresponding to Brazilian Patent Application PI 0002693-0 still unexamined, which are incorporated herein by reference.

The benzodiazepines exhibit anxiolytic, tranquilizers, hypnotics and muscle relaxant effects causing amnesia and psychomotor changes and acting primarily on the limbic system. It reduces the functional activity of the hypothalamus and the cortex through the potentiation of gamma-aminobutyric acid (GABA), an inhibitory neurotransmitter of the central nervous system. Diazepam and midazolam are the two most commonly used agents accordingly, being midazolam a drug of shorter half-life than diazepam and with greater hypnotic potency.

Tramadol is a synthetic analogue of codeine and has analgesia compared to the one produced by morphine when administered at equipotent doses. Its use in veterinary medicine is still very limited, but recent research demonstrates its efficacy and safety in dogs. For example, at a dose of 2 mg/kg intravenously (slow), in a prophylactic way, tramadol is shown to provide analgesia similar to that produced by morphine after ovariosalpingohisterectomy in female dogs, reducing the expired concentration of isoflurane and modulating neuroendocrine response to pain, reducing the peaks of catecholamine and cortisol.

Co-administration of ketamine and benzodiazepines represent one of the most widely used associations for anesthesia and premedication in small animals, providing security, minimal depressant effects, and rapid recovery from anesthesia.

However, it is common knowledge in the art that veterinary anesthesiology continuously aims at pharmacological contention to establish gory treatments or not allowing safe and durable manipulations, eliminating general anesthesia without changing greatly the physiological parameters.

### Summary of the Invention

Through the above knowledge and many experiments carried out by Depositor it was found that the use of ketamine in combination with midazolam and tramadol, according to the present invention, allows to obtain a satisfactory sedation or anesthesia, with greater practicality and economy in choosing a safe and effective sedation and analgesia protocol for pets by the administration of a single product, promoting cardiovascular stability and lasting sedation and analgesia.

Particularly, the isomer S-ketamine (+) is preferred due to advantages with respect to the formulations of racemic ketamine.

Furthermore, the association according to the present invention provides greater cardiovascular, biochemistry and hemogasimetric stability, which means proportionately greater safety in animals during anesthesia or during recovery from the anesthesia, combined with a better quality of sedation, muscle relaxation and analgesia.

### Detailed Description of the Invention

The present invention therefore aims to provide a new anesthetic association for veterinary use comprising at least the active compounds ketamine, particularly the isomer S-ketamine(+) (Compound A), midazolam (Compound B), and tramadol (Compound C)

According to the present invention, a new anesthetic association is formulated in true solution for parenteral use, and is suitable for surgical cases as the agent of pre-anesthetic medication, providing preoperative sedation in lower doses of general anesthetics, besides analgesia before, during and after surgery.

Preferably, but not limited to, the anesthetic combination according to the present invention comprises the active ingredients ketamine, particularly the isomer ketamine-(S+), midazolam and tramadol, in the proportions of 10:1:2, respectively, which may be present in associations as free base or one or more pharmaceutically acceptable salts, such as, for example, maleates and / or hydrochlorides.

Yet another objective of the present invention is to provide a new anesthetic composition comprising as active ingredients at least the compounds ketamine, particularly the isomer ketamine-(S+), midazolam and tramadol, carriers and adjuvants pharmaceutically acceptable for veterinary use.

The active compounds may be present in the composition according to the present invention as free base or one or more pharmaceutically acceptable salts.

Thus, in one non-limitative embodiment of the invention, the composition includes the active ingredient ketamine, particularly the isomer ketamine-S(+) as hydrochloride, the active ingredient midazolam as maleate and/or hydrochloride and the active ingredient tramadol as hydrochloride.

The anesthetic compositions according to the present invention comprise at least 3% w/v of the association of the active ingredients ketamine, particularly the isomer ketamine-(S+), midazolam and tramadol, and carriers and adjuvants pharmaceutically acceptable in veterinary medicine.

Preferably, the anesthetic compositions according to the present invention comprise the active ingredient ketamine, particularly the isomer ketamine-(S+) in proportions ranging from 2.5 to 20% w/v, the active ingredient midazolam in proportions ranging from 0.25 to 2% w/v, the active ingredient tradamol in proportions ranging from 0.25 to 4% w/v, and carriers and adjuvants pharmaceutically acceptable in veterinary medicine.

The carriers and adjuvants used in the compositions object of the present invention may be any one or more selected from those pharmaceutically acceptable in veterinary medicine. However, preferably, it can be used one or more selected from the group consisting of:

- Isotonizing agents: inorganic salts of sodium (NaCl), potassium (KCl), sugars such as dextrose and lactose, polyalcohols such as glycerin, propylene glycol and polyethylene glycol, which have the function of the adjustment of the osmolarity of the solution to avoid plasmolysis.

- Preservatives and/or antimicrobial preservatives: methylparabens, propylparaben, ethylparabens, benzyl alcohol, benzethonium chloride, benzalkonium chloride, phenols, cresols, chlorobutanols, thimerosal, phenylmercuric compounds.

- Antioxidants and /or reducers: bisulfites, sulfites, metabisulfites, thiosulphates, propyl gallate, NDGA, EDTA, thioureas, monotiossorbitol, citric acid, tartaric acid, BHT, BHA, ascorbic acid and tocopherols.

- Cosolvents: pyrrolidones and derivatives thereof, glycols and polyglycols, polyethylene glycols, triacetin, ethers and esters of fatty acids, alcohols, glycerols and amides.

As said, besides those mentioned above, other isotonizing, preservatives, antioxidants, cosolvents, as well as other carriers and adjuvants may also be formulated together with the ingredients of the anesthetic association object of the present invention.

Table 1 below, which should not be construed as limiting for the purposes of the present invention, illustrates a series of analgesic compositions formulated according to the teachings above.

**TABLE 1**

| Compound A | Compound B | Compound C | Inert |
|---|---|---|---|
| 10% w/v | 1% w/v | 2% w/v | 87% w/v |
| 5% w/v | 0.5% w/v | 1% w/v | 93.5% w/v |
| 2.5% w/v | 0.25% w/v | 0.5% w/v | 96.75% w/v |
| 15% w/v | 1.5% w/v | 3% w/v | 80.5% w/v |
| 20% w/v | 2% w/v | 4% w/v | 74% w/v |

| | | | |
|---|---|---|---|
| Compound A = ketamine, particularly the isomer ketamine-(S+) Compound B = midazolam Compound C = tramadol | | | |

The invention also relates to an anesthetic drug parenterally administrated, preferably intramuscularly, containing sufficient amounts of anesthetic association so as to provide the animal with dosages of the active ingredients ketamine, particularly the isomer ketamine-(S+) (Compound A) ranging from 5.0 to 50.0 mg/kg of body weight, midazolam (Compound B) ranging from 0.5 to 5.0 mg/kg of body weight and tramadol (Compound c) ranging from 1.0 to 10, 0 mg/kg of body weight. Preferably, the dosage should guarantee the delivery of 10 mg/kg of body weight of Compound A, 1 mg/kg of body weight of Compound B and 2 mg/kg of body weight of Compound C.

Yet according to an important aspect of the present invention, the administration intramuscularly of a composition comprising as active ingredients 100 mg/ml of ketamine, particularly the isomer ketamine-S(+), 10 mg/ml of midazolam and 20 mg/mL of tramadol, would be indicated for animals in the following situations:

a) as a single agent of the pre-anesthetic medication in the volume of 1 ml per each 10 kg of body weight, intramuscularly, thus reducing the dose of the inducing agent and the requirement of general anesthetics;

b) as a sedative for diagnostic tests (X-rays, ultrasound, biopsies, material collection);

c) as a sedative for performing minimally invasive procedures such as cleaning wounds, changing dressings, small skin sutures associated with local anesthetics, placement and removal of bandages and splints;

d) as a pre-emptive analgesia, reducing the requirement of analgesics trans and postoperative;

e) as a commonly used sedative for clinical, diagnostic or laboratory examinations in excited and/or aggressive animals;

f) associated with spinal anesthesia for carrying out minor surgical interventions such as orchiectomy, skin sutures, wound debridement, among others.

### Experimental Tests

Several experimental studies of pharmacological evaluation of the anesthetic association according to the present invention were conducted in comparison to a marketed product whose active ingredient is an association of tiletamine and zolazepam.

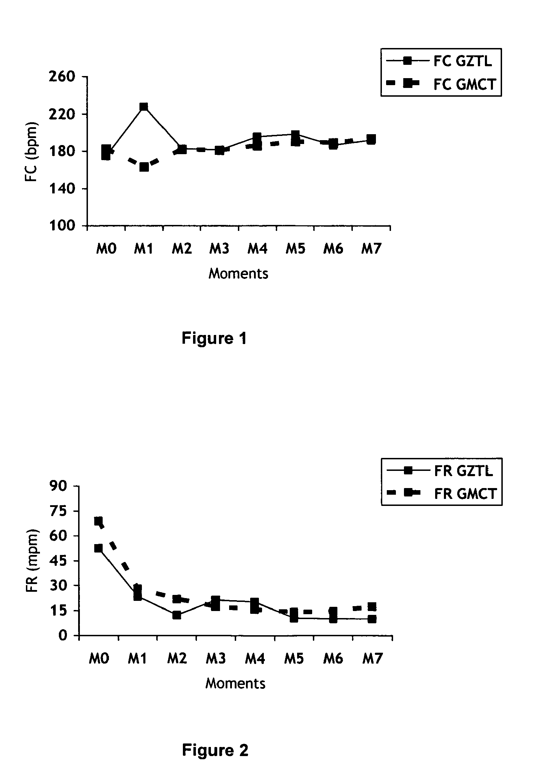

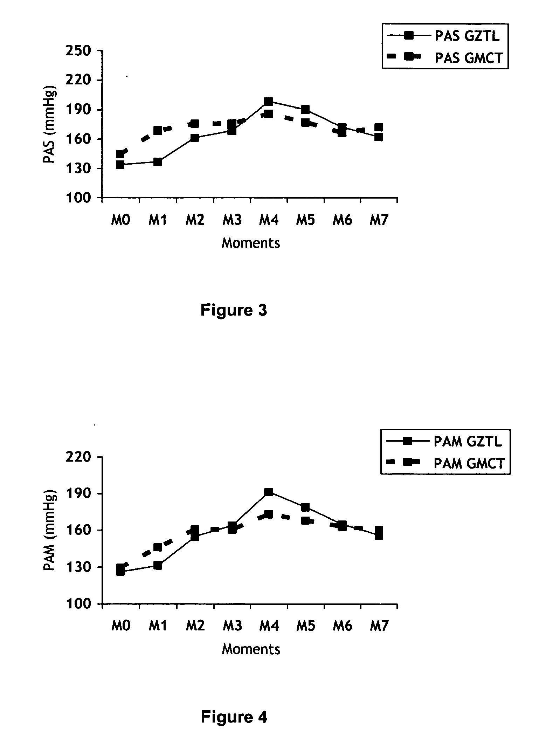

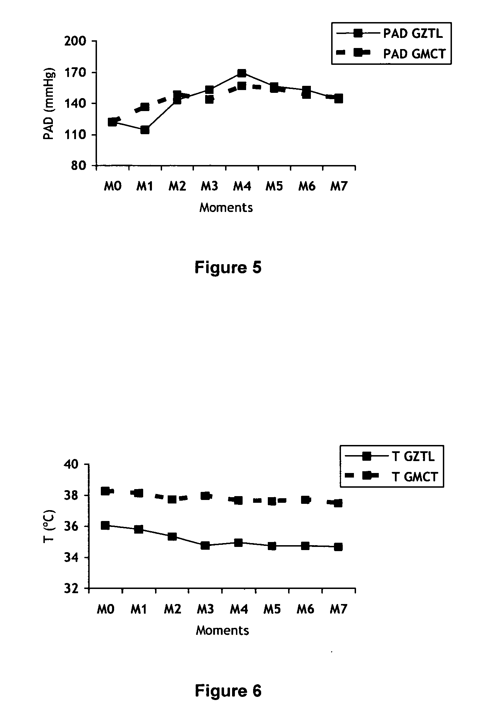

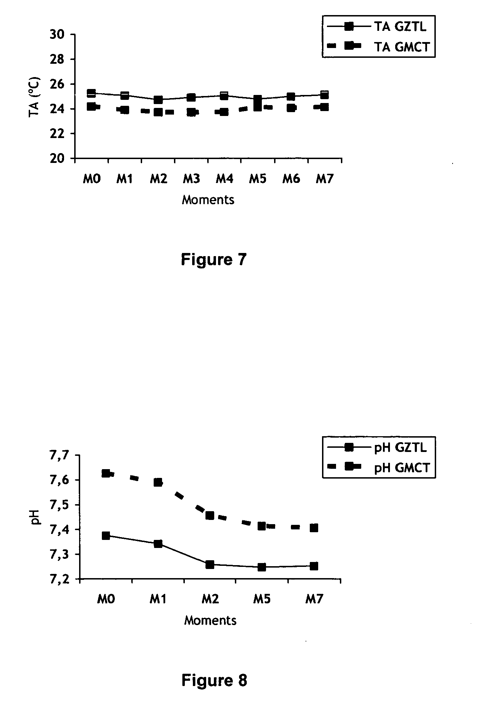

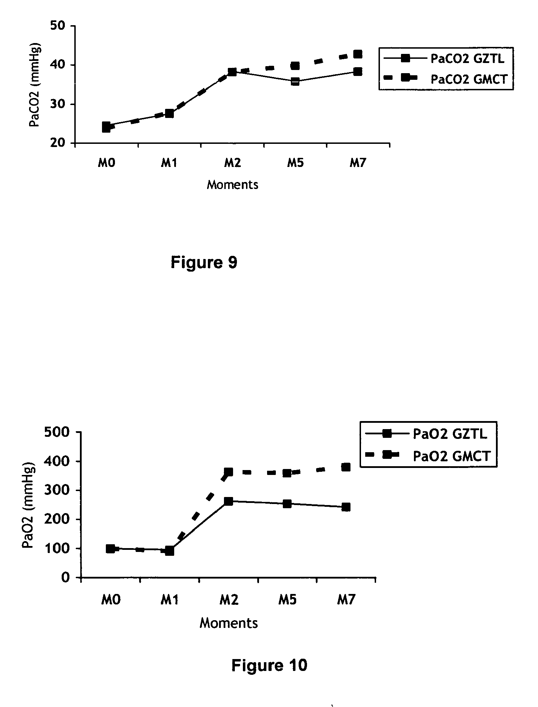

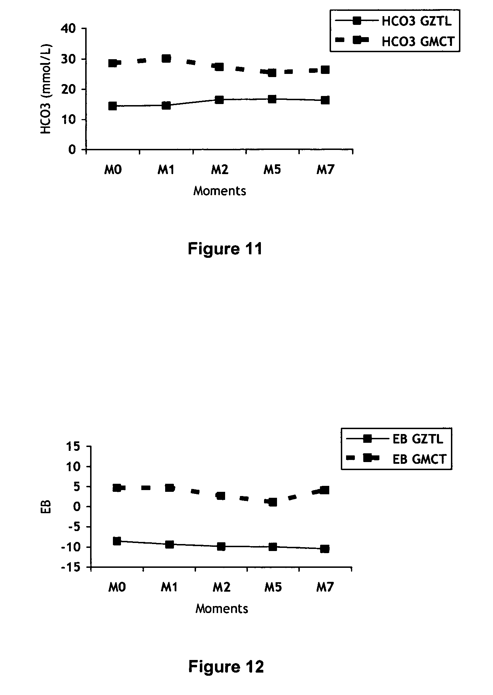

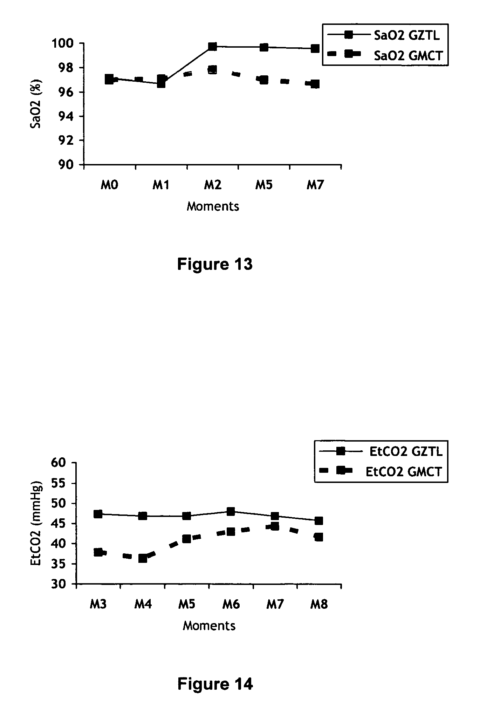

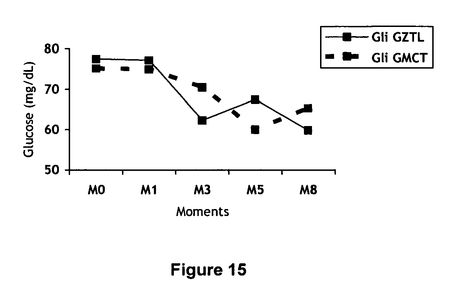

The studies were conducted with female cats after pre-anesthetic medication with medication of the prior art tiletamine/zolazepam (GZTL) and with the drug of the present invention ketamine/midazolam/tramadol (GMCT), and subjected to continuous infusion of propofol in 1% microemulsion to perform ovariosalpingohisterectomy. These studies measured the mean values and standard deviation of heart rate (HR), respiratory rate (RR), rectal temperature (RT), systolic blood pressure (SBP), mean (MAP) and diastolic (DBP), glucose and blood gas analysis (arterial pressure of CO2 - PaCO2, arterial pressure of 02 - Pa02, hydrogen potential - pH, bicarbonate ions - HC03-, CO2 concentration at the end of expiration - EtCO2 and base excess - EB). The results were tabulated and shown in Figures 1 to 15 hereinafter discussed.

The results of the moments M2 and M3 present in the study methodology are shown in the graphs only in M3 because in the assessments both moments ended up being assessed together. Thus, said graphs show that:

### Figure 1:

The FC in the Invention Group did not change statistically in relation to the baseline during the study. However, in the Prior Art Group the FC has raised 15 minutes after pre-anesthetic medication (M1), differing both from the baseline and between groups (greater stability in the FC in the Invention Group).

### Figure 2:

Both in the Invention Group and in the Prior Art Group there was statistical difference for the FR from M1 compared to the baseline, indicating a decrease caused by the administration of the Invention and the Prior Art, respectively. Between groups, during the same experimental time, there was no statistical difference. By viewing the graph, the Invention Group showed a lower decrease of the FR values, as well as improved stability during the evaluation period.

### Figure 3:

In the Invention Group there was an increase of the SBP compared to the baseline in M1, indicating an increase in the SBP, and in the Prior Art Group there was an increase in the SBP after M3 compared to the baseline, indicating an increase in pressure caused after the anesthetic induction and not after the PAM. In the evaluation between groups, there was a difference in M1, with greater pressure in the Invention Group compared to the Prior Art Group (caused by the administration of the Invention).

### Figure 4:

In the Invention Group, PAM was greater from M1 (compared to the baseline), indicating that the administration of the Invention increased PAM of the animals. For the Prior Art Group there was an increase of PAM from M2, indicating an increase in MAP caused after the anesthetic induction. There was no difference among the groups, indicating a similar increase in the pressure between them.

### Figure 5:

In the Invention Group there was no increase of PAD after M1 compared to the baseline, indicating an increase in PAD after the administration of the Invention. For the Prior Art Group there was no difference of PAD compared to the baseline. There was no difference in DBP among the groups. The group receiving the Invention showed increased arterial pressures from M1, indicating an increase of it caused by the administration of the Invention. There is evidence that ketamine has direct adrenergic effect by directly binding to α and β-adrenergic receptors. These observations suggest that the sympathomimetic effect of ketamine is due to the combination of sympathetic nervous system stimulation centrally mediated as a possible effect of ketamine on blocking the reuptake of catecholamines, causing it to increase the blood pressure after its administration.

### Figure 6:

For the Invention Group there was no difference of rectal temperature during the procedure. In the Prior Art Group there was no difference compared to the baseline from M3 (moment of aperture of the abdominal cavity). There were differences between groups only in M3, namely, smaller variation and less decrease of body temperature of the animals receiving the Invention.

### Figure 7:

In both groups there was no difference between the times within the same group. Among groups, at the same moment, room temperature was statistically lower in the Invention Group between M0 and M8, except in M5. Despite the difference in room temperature, the animals that had received the Invention showed higher average body temperature during the procedure, namely, higher thermal stability.

### Figure 8:

In both groups there was a pH difference compared to the baseline from M2, caused by anesthetic induction or by supplemental 02 performed during the procedure. There were differences among groups from the baseline to the last assessment. The groups had similar pH variation.

### Figure 9:

In the Invention Group there was a difference in PaCO2 (increase) from M1 compared to the baseline, caused after the administration of the Invention. In the Prior Art Group there was a difference from M2 (increase) compared to the baseline. There were differences among the groups in M7, in which the Invention Group remained higher than the Prior Art Group. Despite the difference among groups, the values remained within physiological limits.

### Figure 10:

In both groups there was a difference compared to the baseline from M2, caused by supplemental 02 after the anesthetic induction. There were differences between groups from the beginning of supplemental 02 in M2. The animals that received the Invention showed higher values of arterial oxygen pressure during the anesthetic procedure.

### Figure 11:

In the Invention Group there was a difference in M5 compared to the baseline (decrease), and in the Prior Art Group there was a difference from M2 compared to the baseline (increase).

### Figure 12:

Despite the variation between moments observed in both groups, i.e., a decrease followed by an increase in the Invention Group and a decrease in the Prior Art Group. Animals receiving the Invention showed higher excess values of arterial base compared to the animals that received the Prior Art throughout the study.

### Figure 13:

Despite the variation observed among treatments during the study, the values obtained remained within the established as physiological for the species, which is above 95%.

### Figure 14:

There was no statistical difference among the groups at the same moment, as well as between moments in the same group, indicating similarity of results and stability.

### Figure 15:

There was no statistical difference among the groups at the same moment, as well as between moments in the same group, indicating similarity of results and stability.

Thus, the studies showed that the degree of sedation in the Invention Group is considered optimal in 100% of the animals, whereas in the Prior Art Group this index was achieved in only 66% of the animals.

The animals receiving the association of the Invention as a pre-anesthetic medication (PAM) showed cardiorespiratory and hemogasimetric stability, and a better degree of sedation compared to the animals that received the association of the Prior Art as PAM.

## Claims

1. An anesthetic association for veterinary use, intended for use in surgical, pre-surgical and other procedures which require sedation, tranquilization, chemical restraint and pre-anesthetic medication, **characterized in that** it comprises the association of at least the active compounds ketamine, midazolam and tramadol.

2. An association according to claim 1, **characterized in that** the active compounds midazolam and tramadol are present as free base or a pharmaceutically acceptable salt.

3. An association according to claim 2, **characterized in that** the pharmaceutically acceptable salt is a maleate, a hydrochloride or mixtures thereof.

4. An association according to any one of claims 1 to 3, **characterized in that** the compounds ketamine, midazolam and tramadol are present in a ratio of 10:1:2, respectively.

5. An association according to any one of claims 1 to 4, **characterized in that** the active compounds ketamine, midazolam and tramadol are formulated in true solution for parenteral use.

6. An association according to any one of claims 1 to 5, **characterized in that** the active compound ketamine is the isomer ketamine-(S+).

7. An anesthetic composition for veterinary use, intended for use in surgical, pre-surgical and other procedures which require sedation, tranquilization, chemical restraint and pre-anesthetic medication, **characterized in that** it comprises as active ingredients the association of the compounds ketamine, midazolam and tramadol in an amount of at least 3% w/v and pharmaceutically acceptable carriers and adjuvants.

8. A composition according to claim 7, **characterized in that** the active compounds ketamine, midazolam and tramadol are present in the composition as free base or one or more pharmaceutically acceptable salt.

9. A composition according to claim 8, **characterized in that** the active ingredient ketamine is provided as hydrochloride, the active ingredient midazolam is provided as maleate and/or hydrochloride and the active ingredient tramadol is provided as hydrochloride.

10. A composition according to claim 7, **characterized in that** the active ingredient ketamine in proportions ranging from 2.5 to 20% w/v, the active ingredient midazolam in proportions ranging from 0.25 to 2% w/v, and the active ingredient tradamol in proportions ranging from 0.5 to 4% w/v.

11. A composition according to any one of claims 7 to 10, **characterized in that** the active compound ketamine is the isomer ketamine-(S+).

12. A composition according to claim 7, **characterized in that** the carriers and adjuvants are isotonizing compounds, preservatives, antioxidants and cosolvents.

13. A composition according to claim 12, **characterized in that** the carriers and adjuvants are one or more compounds selected from the group consisting of:
- isotonizing agents: inorganic salts of sodium (NaCl), potassium (KCl), sugars such as dextrose and lactose, polyalcohols such as glycerin, propylene glycol and polyethylene glycol, which have the function of the adjustment of the osmolarity of the solution to avoid plasmolysis.
- preservatives and/or antimicrobial preservatives: methylparabens, propylparaben, ethylparabens, benzyl alcohol, benzethonium chloride, benzalkonium chloride, phenols, cresols, chlorobutanols, thimerosal, phenylmercuric compounds.
- antioxidants and /or reducers: bisulfites, sulfites, metabisulfites, thiosulphates, propyl gallate, NDGA, EDTA, thioureas, monotiossorbitol, citric acid, tartaric acid, BHT, BHA, ascorbic acid and tocopherols.
- cosolvents: pyrrolidones and derivatives thereof, glycols and polyglycols, polyethylene glycols, triacetin, ethers and esters of fatty acids, alcohols, glycerols and amides.

14. An anesthetic drug for veterinary use, intended for use in surgical, pre-surgical and other procedures which require sedation, tranquilization, chemical restraint and pre-anesthetic medication, and for parenteral administration **characterized in that** it comprises sufficient amounts to provide the animal with dosages of the active ingredients ketamine, ranging from 5.0 to 50.0 mg/kg of body weight, midazolam ranging from 0.5 to 5.0 mg/kg of body weight and tramadol ranging from 1.0 to 10, 0 mg/kg of body weight.

15. A drug according to claim 14, **characterized in that** the dosages ensure the delivery of 10 mg/kg of body weight of ketamine, 1 mg/kg of body weight of midazolam and 2 mg/kg of body weight of tramadol.

16. A drug according to any one of claims 14 to 15, **characterized in that** the active compound ketamine is the isomer ketamine-(S+).
